# EUROPEAN PATENT SPECIFICATION

(11) **EP 2 623 048 B1**
(45) Date of publication and mention of the grant of the patent: **30.09.2015**
(21) Application number: 11828040.3
(22) Date of filing: 26.08.2011
(51) Int. Cl.: A61B 17/16, B23B 31/10

(54) **DRILL BIT LOCKING DEVICE AND DRILL BIT**
VERRIEGELUNGSVORRICHTUNG FÜR EINEN BOHRMEISSEL UND BOHRMEISSEL
DISPOSITIF DE VERROUILLAGE D'UN OUTIL DE FORAGE ET OUTIL DE FORAGE

(30) Priority: 30.09.2010 CN 201010298086
(43) Date of publication of application: 07.08.2013
(73) Proprietor: CHONGQING RUNZE PHARMACEUTICAL CO., LTD., Yubei District Chongqing 401120 (CN)
(72) Inventor: YE, Lei, Chongqing 401120 (CN); ZHOU, Jian, Chongqing 401120 (CN); FENG, Hua, Chongqing 401120 (CN); LI, Fei, Chongqing 401120 (CN); ZHU, Hengyang, Chongqing 401120 (CN); LI, Congxiao, Chongqing 401120 (CN)
(74) Representative: Boden, Keith McMurray
(86) International application number: PCT/CN2011/078964
(87) International publication number: WO 2012/041144

(56) References cited:
- CN-A- 101 926 670
- CN-A- 101 926 671
- CN-A- 101 972 157
- CN-U- 201 492 470
- CN-U- 201 798 776
- CN-U- 201 798 777
- CN-U- 201 798 778
- CN-Y- 201 067 895
- US-A- 5 222 956
- US-A- 5 398 946
- US-A- 5 490 683
- US-A1- 2004 081 523

## Description

### Field of the Invention

The present invention relates to a drill head locking device according to claim 1, that can be used with a bone drill motor for use in surgery.

### Description of the Prior Art

In surgery, the cranial drill, milling cutter and abrasion drill are tools commonly used for craniotomy. The cranial drill comprises a main machine, a reducer, a locking seat and a drill head. The drill head comprises cutting edge in the front part and a transmission rod in the rear part. The drill has a transmission rod drill head, and the locking seat is threadedly connected to the front part of the reducer, having the drill head passing through the locking seat and coupled with the power output of the reducer. The locking seat effects tight locking of the transmission rod to prevent the detachment of the drill head in operation. The prior locking device utilizing a trip to effect locking is inconvenient for mounting and difficult for use.

US-A-5490683 discloses an apparatus for coupling a driving shaft and a mating tool shaft to transmit rotational forces and axial tension and compression forces betweem the shafts.

### Summary of the Invention

The object of the present invention is to provide a drill head locking device and a drill head that are convenient for use.

The object of the present invention is accomplished by adopting the following scheme.

A drill head locking device, wherein the device comprises: a flange having a through hole, bearings arranged on the inner wall of a small cylinder on the upside of the flange and a supporting member for supporting the bearings, a locking sleeve arranged over the flange, a first protrusion arranged on the inner wall of the upper part of the locking sleeve, the inner wall of the lower part of the locking sleeve having a second protrusion. A spring is arranged between the second protrusion and an upper bottom surface of the flange. A pressing plate is threadedly connected to the outer wall of the top part of the small cylinder on the upside of the flange and presses against the first protrusion. Holes of different sizes are arranged on the inner wall of the small cylinder in the corresponding positions to the second protrusion and on the supporting member, thus forming a stepped-hole which is arranged with a steel ball therein.

In order to transfer uniform force to the transmission rod, the bearings are numbered to be two and are respectively positioned below the outer wall and above the upper bottom surface.

In order to ensure the bearings and the pressing member are secured, the upper bottom surface is arranged with a pressing member threadedly connected to the inner wall of the upper bottom surface for tightly abutting the bearings and the supporting member.

In order to ensure smooth movement of the transmission rod, the bearings are numbered to be two and are respectively positioned below the outer wall and above the pressing member.

In order to facilitate connecting the reducer, the inner wall of the big cylinder on the underside of the flange is provided with inner threads.

A drill head mating the noted drill locking device, comprises a cutting edge in the front part and a transmission rod in the rear part, wherein a groove is provided on the transmission rod.

By adopting this structure, when the transmission rod is to be inserted, one only has to press down the locking sleeve, then the second protrusion moves away from the steel balls which engages the rod body of the transmission rod and rolls toward outside. When the transmission rod reaches the designated position, one releases the locking sleeve which moves up upon the effect of the spring, the second protrusion engages the steel ball and moves inwardly to snap into the snap-slot on the transmission rod which is thus secured. In such a locking manner, the locking and loosening operations are easy and convenient to apply, providing a firm securing effect when in locking status and improved safety.

### Brief Description of the Drawings

Figure 1 is a structural schematic view of an embodiment of the present invention;
Figure 2 is a structural schematic view of a drill head in an embodiment of the present invention.

### Detailed Description of the Preferred Embodiments

Hereinafter further description will be made by incorporating figures and embodiments to illustrate the present invention.

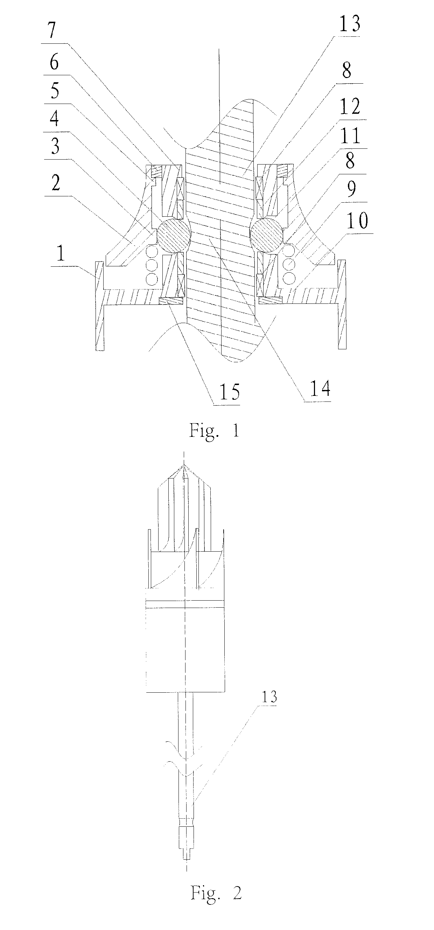

As can be seen from Figure 1 to Figure 2:
A drill head, comprises a cutting edge in the front part and a transmission rod in the rear part, with a groove 14 provided on the transmission rod 13.
A drill locking device comprises a flange 1 with a through hole. The inner wall of the small cylinder on the upside of the flange is arranged with bearings 8 thereon, which are numbered to be two and respectively positioned below the outer wall 7 and above the pressing member 15. A supporting member 12 is arranged between the two bearings 8. A pressing member 15 is provided on the upper bottom surface 10 and is threadedly connected to the inner wall of the upper bottom surface, for tightly pressing against the bearings 8 and the supporting member 12. The two bearings may effectively ensure the smooth rotation of the transmission rod to produce lesser heat, and the existence of the supporting member 12 enhances to the firmness between the bearings to reduce the likelihood of detachment.

A locking sleeve 2 is arranged over the flange, with a first protrusion 5 arranged on the inner wall of the upper part of the locking sleeve 2, and a second protrusion 3 arranged on the inner wall of the lower part of the locking sleeve 2. A spring 9 is arranged between the second protrusion 3 and an upper bottom surface 10 of the flange. A pressing plate 6 is threadedly connected on the outer wall 7 of the top part of the small cylinder on the upside of the flange and presses against the first protrusion 5. Holes of different sizes are arranged on the inner wall of the small cylinder in the corresponding positions to the second protrusion 3 and the supporting member 12, thus forming a stepped-hole 11 which is arranged with a steel ball 4 therein. The diameter of the hole on the inner wall of the small cylinder is greater than that of the steel ball 4, and the diameter of the hole on the supporting member 12 is less than that of the steel ball 4.

A transmission rod 13 of the drill head is provided with a groove 14 in the corresponding position to the stepped hole 11. The inner wall of the big cylinder on the underside of the flange 1 is provided with inner threads. In this embodiment, the number of the stepped holes 11 and the steel balls 4 are both counted to be two respectively, and as a variant, the number of the steel balls and the stepped holes may be set to be three, so that an improved locking effect is offered.

The techniques described herein are exemplary, and should not be construed as implying any particular limitation on the present disclosure. It should be understood that various alternatives, combinations and modifications could be devised by those skilled in the art without departing from the technical scheme content of the present invention. The present disclosure is intended to embrace all alternatives, modifications and variances that fall within the scope of the appended claims.

## Claims

1. A drill head locking device for surgical use, wherein the device comprises: a flange (1) having a through hole, bearings (8) arranged on the inner wall of a small cylinder on the upside of the flange and a supporting member (12) for supporting the bearings (8), a locking sleeve (2) arranged over the flange, a first protrusion (5) arranged on the inner wall of the upper part of the locking sleeve (2), the inner wall of the lower part of the locking sleeve (2) having a second protrusion (3) arranged thereon; a spring (9) is arranged between the second protrusion (3) and an upper bottom surface (10) of the flange; a pressing plate (6) is threadedly connected to an outer wall (7) of the top part of the small cylinder on the upside of the flange and presses against the first protrusion (5); holes of different sizes are arranged on the inner wall of the small cylinder in the corresponding positions to the second protrusion (3) and on the supporting member (12), forming a stepped-hole (11) which is arranged with a steel ball (4) therein.

2. The drill head locking device according to claim 1, **characterized in that**, the upper bottom surface (10) is arranged with a pressing member (15) threadedly connected to the inner wall of the upper bottom surface for tightly pressing against the bearings (8) and the supporting member (12).

3. The drill head locking device according to claim 2, **characterized in that**, the number of the bearings (8) are two and respectively positioned below the outer wall (7) and above the pressing member (15).

4. The drill head locking device according to any of claims from 1 to 3, **characterized in that**, the inner wall of the big cylinder on the underside of the flange (1) is provided with inner threads.

5. A combination of the drill head locking device according to any of claims 1 to 4 and a drill head mating threwith, wherein the drill head comprises a cutting edge in a front part and a transmission rod (13) in a rear part, and a groove (14) is provided on the transmission rod (13).

## Patentansprüche

1. Bohrkopf-Sperreinrichtung zur Verwendung bei einer Operation, worin die Einrichtung umfasst: eine Flansch (1) mit einem Durchgangsloch, Lager (8), die an einer Innenwand eines kleinen Zylinders auf der Oberseite der Flansch angeordnet sind, und ein Trägerelement (12) zum Tragen der Lager (8), eine Sperrbuchse (2), die über der Flansch angeordnet ist, einen ersten Vorsprung (5), der an einer Innenwand des oberen Teils der Sperrbuchse (2) angeordnet ist, worin die Innenwand des unteren Teils der Sperrbuchse (2) einen zweiten Vorsprung (3) aufweist; worin eine Feder (9) zwischen dem zweiten Vorsprung (3) und einer oberen Bodenfläche (10) der Flansch angeordnet ist; worin eine Pressplatte (6) mit einer Außenwand (7) des oberen Teils des kleinen Zylinders auf der Oberseite der Flansch über ein Gewinde verbunden ist und gegen den ersten Vorsprung (5) drückt; worin Löcher unterschiedlicher Größe auf der Innenwand des kleinen Zylinders in den jeweiligen Position des zweiten Vorsprungs (3) und auf den Trägerelement (12) angeordnet sind, die ein abgestuftes Loch (11) ausbilden, das mit einer Stahlkugel darin vorgesehen ist.

2. Bohrkopf-Sperreinrichtung nach Anspruch 1, **dadurch gekennzeichnet, dass** die obere Bodenfläche (10) mit einem Presselement (15) angeordnet ist, das mit der Innenwand der oberen Bodenfläche zur festen Pressung gegen die Lager (8) und das Trägerelement (12) über ein Gewinde verbunden ist.

3. Bohrkopf-Sperreinrichtung nach Anspruch 2, **dadurch gekennzeichnet, dass** die Anzahl der Lager (8) zwei ist, die jeweils unter der äußeren Wand (7) und über dem Presselement (15) angeordnet sind.

4. Bohrkopf-Sperreinrichtung nach einem der Ansprüche 1 bis 3, **dadurch gekennzeichnet, dass** die Innenwand des großen Zylinders auf der Unterseite der Flansch (1) mit Innengewinden versehen ist.

5. Kombination der Bohrkopf-Sperreinrichtung nach einem der Ansprüche 1 bis 4 und eines dazu passenden Bohrkopfs, worin der Bohrkopf in einem vorderen Bereich eine Schneidekante und in einem hinteren Bereich eine Übertragungsstange (13) umfasst, worin auf der Übertragungsstange (13) eine Vertiefung (14) vorgesehen ist.

## Revendications

1. Dispositif de verrouillage de tête de trépan à usage chirurgical **caractérisé en ce que** le dispositif comprend : un collerette (1) ayant un trou traversant, des paliers (8) ménagés sur la paroi intérieure d'un petit cylindre sur la partie supérieure de la collerette et un membre de support (12) pour supporter les paliers, un manchon de verrouillage (2) ménagé au-dessus de la collerette, une première saillie (5) ménagée sur la paroi intérieure de la partie supérieure du manchon de verrouillage (2), la paroi intérieure de la partie inférieure du manchon de verrouillage (2) étant pourvue d'une deuxième saillie (3); un ressort (9) ménagé entre la deuxième saillie (3) et une surface de fond supérieure (10) de la collerette; une plaque (6) de pression qui est reliée par filetage à une paroi extérieure (7) de la partie sommitale du petit cylindre sur la partie supérieure de la collerette et qui presse contre la première saillie (5), des trous de différentes taille ménagés sur la paroi intérieure du petit cylindre dans les positions correspondant à la deuxième saillie (3), et sur le membre de support (12), formant un alésage étagé (11) qui est configuré en recevant une bille d'acier (14).

2. Dispositif de verrouillage de tête de trépan selon la revendication 1, **caractérisé en ce que** la surface de fond supérieure (10) est pourvue d'un membre de pression (15) relié par filetage à la paroi intérieure de la surface de fond supérieure pour presser de manière ajustée contre les paliers (8) et le membre de support (12).

3. Dispositif de verrouillage de tête de trépan selon la revendication 2, **caractérisé en ce que** les paliers (8) sont au nombre de deux et disposés respectivement sous la paroi extérieure (7) et au-dessus du membre de pression (15).

4. Dispositif de verrouillage de tête de trépan selon l'une quelconque des revendications 1 à 3, **caractérisé en ce que** la paroi intérieure du grand cylindre sur la partie inférieure de la collerette (1) est pourvu d'un taraudage.

5. Combinaison du dispositif de verrouillage de tête de trépan selon l'une quelconque des revendications 1 à 4 et d'une tête de trépan assortie, dans laquelle la tête de trépan comprend un bord coupant dans une partie frontale et une tige de transmission (13) dans une partie arrière, et une gorge pratiquée sur la tige de transmission.
